# EUROPEAN PATENT APPLICATION

(11) **EP 4 613 307 A1**
(43) Date of publication of application: **10.09.2025**
(21) Application number: 23886194.2
(22) Date of filing: 31.10.2023
(51) Int. Cl.: A61M 15/00

(54) **INHALER**

(30) Priority: 02.11.2022 KR 20220144248
(71) Applicant: KT&G Corporation, Daedeok-gu Daejeon 34337 (KR)
(72) Inventor: KIM, Moon Won, Daejeon 34128 (KR); JUNG, Yong Mi, Daejeon 34128 (KR); SHIN, Jun Won, Daejeon 34128 (KR)
(74) Representative: AWA Sweden AB
(86) International application number: PCT/KR2023/017062
(87) International publication number: WO 2024/096498

(57) **Abstract**

An inhaler according to an embodiment comprises: a main body that has a first surface and a second surface facing the first surface and provided with a mouthpiece and has, on the inside, a longitudinal channel going from the first surface toward the second surface; a cartridge that is coupled to the first surface of the main body and in communication with the channel and contains an inhalable composition; and a plurality of baffles disposed in the channel, wherein, when a suction force is applied to the mouthpiece, the baffles can limit the particle size of the inhalable composition that is transferred to the mouthpiece.

## Description

### TECHNICAL FIELD

An inhaler is disclosed.

### BACKGROUND ART

In general, an inhaler is a device used to inhale a composition such as a drug or other substance as a liquid or gas through an oral cavity or nasal cavity during an inhalation process. Such an inhaler is equipped with a container that accommodates an inhalable composition, and the composition may be sprayed from the container through a tube connected to the container and finally into the oral cavity or nasal cavity of a user through an intake. In addition to a liquid inhaler and a vapor inhaler, such an inhaler includes a powder inhaler that sprays fine powder of the composition as microparticles and an aerosol inhaler that supplies the composition by aerosolizing the composition.

A dry powder inhaler is intended to transfer a single-dose, metered dose of a pharmacological agent into the body and has been used primarily to treat patients with asthma and chronic obstructive pulmonary disease (COPD). Finely dried powders of 5 to 10 micrometers (µm) or less are used for efficient absorption of the agent into the body. In addition, a determined amount of drug may be filled into a hard capsule for quantitative delivery. Recently, the application fields of dry powder inhalers have expanded, and the scope has expanded to not only disposable or multi-use powder inhalers, but also powder inhalers that inhale medicinal substances, functional substances, nicotine, and other preference products.

The above description is information the inventor(s) acquired during the course of conceiving the present disclosure or already possessed at the time and is not necessarily art publicly known before the present application was filed.

Prior art document: Korea Patent Publication No. 10-1759972

### DISCLOSURE OF THE INVENTION

### TECHNICAL GOALS

An embodiment is to provide an inhaler that prevents leakage of an inhalable composition and prevents introduction of foreign substances.

The technical goals obtainable from the embodiments are not limited to the above-mentioned technical goals, and other unmentioned technical goals may be clearly understood from the following description by one of ordinary skill in the art to which the present disclosure pertains.

### TECHNICAL SOLUTIONS

An inhaler according to an embodiment for achieving the goals includes a main body that includes a first surface, a second surface facing the first surface and having a mouthpiece, and a plurality of side surfaces connecting the first surface to the second surface and has a channel formed inside in a longitudinal direction from the first surface toward the second surface, a cartridge coupled to the first surface of the main body and containing an inhalable composition, and a cartridge cover surrounding the main body and the cartridge and slidably coupled along a side surface of the main body in the longitudinal direction, wherein the cartridge cover may move in the longitudinal direction to open the cartridge or moves in a direction opposite to the longitudinal direction to close the cartridge.

According to an aspect, the inhaler may further include a cartridge door that is arranged between the main body and the cartridge and opens and closes by interworking with the cartridge cover, wherein the cartridge door may connect the cartridge to the channel when the cartridge cover moves in the longitudinal direction, and wherein the cartridge door may isolate the cartridge from the channel when the cartridge cover moves in the direction opposite to the longitudinal direction.

According to an aspect, the cartridge door may include a door member in a flat shape arranged parallel to the first surface inside the main body and a roller member arranged to be in contact with one surface of the door member and changing a direction of the door member to a direction perpendicular to the first surface, wherein the door member may have at least one end connected to the cartridge cover to slide.

According to an aspect, when the cartridge cover moves in the longitudinal direction, the door member may have an area arranged parallel to the first surface that decreases and an area converted in the direction perpendicular to the first surface that increases, and when the cartridge cover moves in the direction opposite to the longitudinal direction, the door member may have the area arranged parallel to the first surface that increases and the area converted in the direction perpendicular to the first surface that decreases.

According to an aspect, the cartridge may include a receiving member that includes a first cartridge surface parallel to one of the plurality of side surfaces and a second cartridge surface perpendicular to the first cartridge surface and in contact with the first surface and contains the inhalable composition therein, an air inlet formed on the first cartridge surface of the receiving member, and an air outlet formed on the second cartridge surface of the receiving member, wherein the air inlet or the air outlet may be opened when the cartridge cover moves in the longitudinal direction and closed when the cartridge cover moves in the direction opposite to the longitudinal direction.

According to an aspect, the air inlet may be formed to extend in the longitudinal direction along the first cartridge surface, and the cartridge cover may control an open area of the air inlet according to a sliding movement distance in the longitudinal direction.

According to an aspect, the air outlet may be formed to extend transversely across the longitudinal direction along the second cartridge surface, and the cartridge cover may control an open area of the air outlet according to a sliding movement distance in the longitudinal direction.

According to an aspect, the cartridge cover may include a cover member arranged to surround the main body and a side surface of the cartridge, and a protruding member protruding inwardly of the cover member and inserted into the side surface of the main body, and a longitudinal length of the cover member may be greater than a longitudinal length of the cartridge.

According to an aspect, the cartridge cover may further include an elastic member arranged between the cover member and the main body, and the elastic member may be compressed when the cover member is pressed in the longitudinal direction and decompressed when the cover member is pressed in the direction opposite to the longitudinal direction.

According to an aspect, a slit extending along the longitudinal direction may be formed on each of two facing side surfaces among the side surfaces the main body, and the cartridge cover may be coupled to the slit and may slide along the slit.

According to an aspect, the cartridge may be detachably attached to the first surface by magnets or in a fitting manner.

### EFFECTS OF THE INVENTION

According to an inhaler according to an embodiment, there is an effect of preventing leakage of an inhalable composition and preventing introduction of foreign substances.

The effects of the inhaler are not limited to the above-mentioned effects, and other unmentioned effects can be clearly understood from the following description by one of ordinary skill in the art to which the present disclosure pertains.

### BRIEF DESCRIPTION OF DRAWINGS

FIG. 1 illustrates an inhaler according to an embodiment.
FIG. 2 illustrates a cross-section of a cartridge cover of an inhaler, according to an embodiment.
FIGS. 3A and 3B illustrate an air inlet of a cartridge that opens according to movement of a cartridge cover.
FIGS. 4A and 4B illustrate a side view of an inhaler according to an embodiment in which a cartridge door operates by interworking with movement of a cartridge cover.
FIGS. 5A and 5B illustrate an air outlet of a cartridge that opens according to movement of the cartridge door of FIGS. 4A and 4B.

The accompanying drawings illustrate preferred embodiments of the present invention and are provided together with the detailed description for better understanding of the technical idea of the present invention. Therefore, the present invention should not be construed as being limited to the embodiments set forth in the drawings.

### BEST MODE FOR CARRYING OUT THE INVENTION

Hereinafter, embodiments are described in detail with reference to the illustrative drawings. Regarding the reference numerals assigned to the components in the drawings, it should be noted that the same components are designated by the same reference numerals, wherever possible, even though they are shown in different drawings. Furthermore, in the following description of the present embodiments, a detailed description of publicly known configurations or functions incorporated herein will be omitted when it is determined that the detailed description obscures the subject matters of the present embodiments.

In addition, the terms first, second, A, B, (a), and (b) may be used to describe components of the embodiments. These terms are used only for the purpose of discriminating one component from another component, and the nature, the sequences, or the orders of the components are not limited by the terms. When one component is described as being "connected", "coupled", or "attached" to another component, it should be understood that one component may be connected or attached directly to the other component, and an intervening component may also be "connected", "coupled", or "attached" to the components.

The same name may be used to describe an element included in the embodiments described above and an element having a common function. Unless otherwise mentioned, the descriptions of the embodiments may be applicable to the following embodiments and thus, duplicated descriptions will be omitted for conciseness.

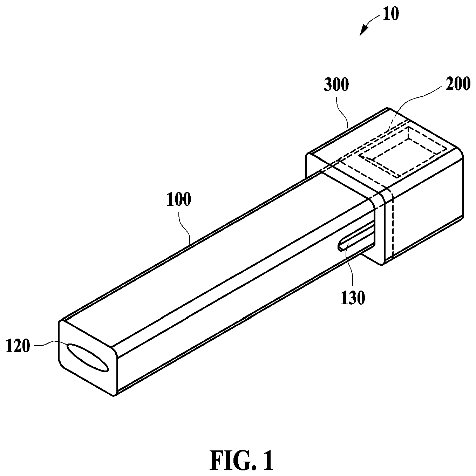

FIG. 1 illustrates an inhaler 10 according to an embodiment.

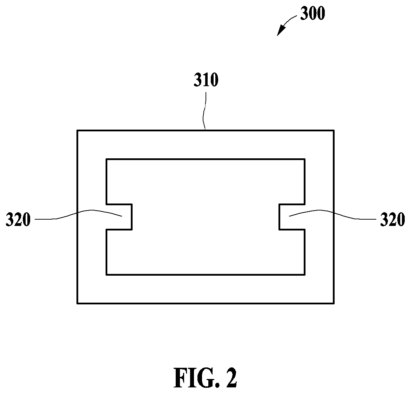

FIG. 2 illustrates a cross-section of a cartridge cover 300 of the inhaler 10, according to an embodiment.

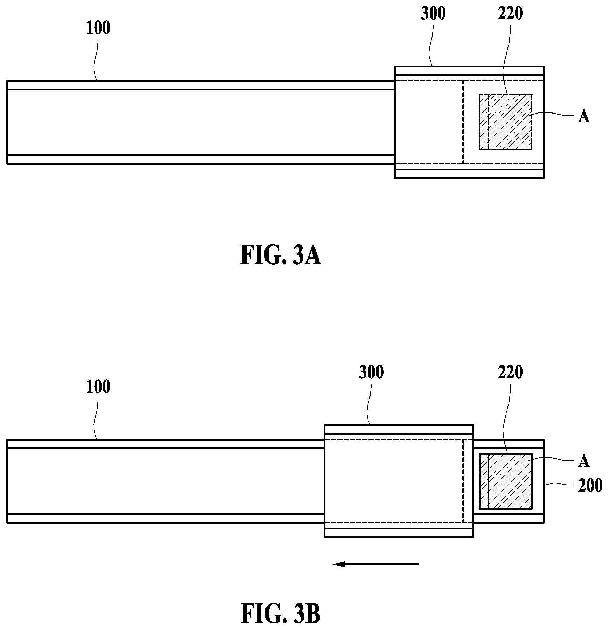

FIGS. 3A and 3B illustrate an air inlet 220 of a cartridge 200 that opens according to movement of the cartridge cover 300.

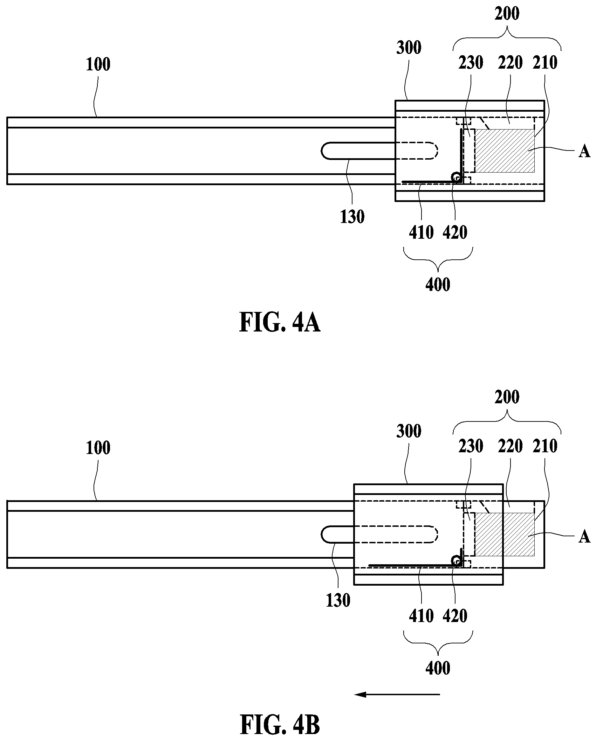

FIGS. 4A and 4B illustrate a side view of the inhaler 10 according to an embodiment in which a cartridge door 400 operates by interworking with movement of the cartridge cover 300.

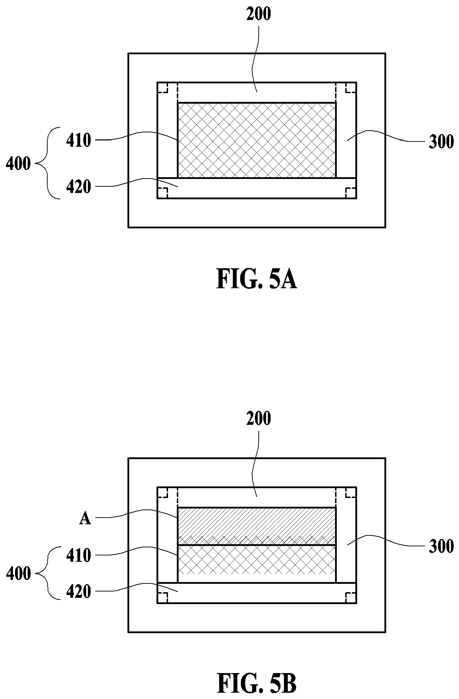

FIGS. 5A and 5B illustrate an air outlet 230 of the cartridge 200 that opens according to movement of the cartridge door 400 of FIGS. 4A and 4B.

Referring to FIG. 1, the inhaler 10 according to an embodiment may include a main body 100, the cartridge 200, and the cartridge cover 300.

The main body 100 may include a first surface, a second surface facing the first surface, and a plurality of side surfaces connecting the first surface to the second surface. The plurality of side surfaces may include, for example, an upper side surface, a lower side surface facing the upper side, and a left side surface and a right side surface that connect the upper side surface to the lower side surface and face each other. The cartridge 200 may be coupled to the first surface. A mouthpiece 120 may be provided on the second surface.

A channel 110 penetrating the first surface and the second surface may be formed inside the main body 100. The channel 110 may be a passage through which an inhalable composition A for delivery to a user is transferred to the mouthpiece 120. Here, the inhalable composition A may be transferred to the mouthpiece 120 along a longitudinal direction from the first surface to the second surface.

The channel 110 may be formed in a spiral shape with an end portion adjacent to the second surface having a spiral shape. Accordingly, a vortex may be generated in the flow of the inhalable composition A.

A cartridge cover 300 may be slidably coupled to an outer surface of the main body 100. Here, the cartridge cover 300 may be coupled to a slit 130 having a shape extending in the longitudinal direction on the side surface of the main body 100. The slit 130 may be formed, for example, on two facing side surfaces of the main body 100. The slit 130 may be formed, for example, on the left side surface and the right side surface of the main body 100. Therefore, the cartridge cover 300 may slidably move along the slit 130.

In addition, the main body 100 may include materials such as polylactic acid (PLA), acrylonitrile butadiene styrene copolymer (ABS), polycarbonate (PC), polypropylene (PP), etc. and may also include other biodegradable (eco-friendly) materials or other metal materials such as stainless steel, aluminum, etc.

The cartridge 200 may be coupled to the first surface of the main body 100. The cartridge 200 may contain the inhalable composition A inside and may be connected internally to the channel 110 of the main body 100. The cartridge 200 may contain one or multiple doses of the inhalable composition A. The inhalable composition A may include, for example, substances such as nicotine, nicotine salt, caffeine, taurine, and vitamins. In addition, the inhalable composition A may include flavor particles, such as natural flavor particles, menthol, and the like. The inhalable composition A may be provided in the form of, for example, dry powder. Here, the dry powder used in the inhaler 10 may have or not have a carrier.

Referring to FIGS. 3A and 3B and FIGS. 4A and 4B, the cartridge 200 may include a receiving member 210, the air inlet 220, and the air outlet 230.

The receiving member 210 may include a first cartridge surface arranged to be parallel or connected to the upper surface of the main body 100 and a second cartridge surface arranged to be perpendicular to the first cartridge surface and in contact with the first surface of the main body 100. The receiving member 210 may accommodate the inhalable composition A therein. Here, the inhalable composition A may be filled in a volume ratio of 30 to 70 percent (%) of an internal space of the receiving member 210. In addition, the inhalable composition A may be filled in a mass of 20 to 70 milligrams (mg) in the receiving member 210.

The air inlet 220 may be formed on the first cartridge surface of the receiving member 210. The air inlet 220 may connect the outside of the inhaler 10 and the inside of the receiving member 210. When the user applies a suction force to the mouthpiece 120, air from the outside may be introduced into the inside of the receiving member 210 containing the inhalable composition A, through the air inlet 220.

The air outlet 230 may be formed on the second cartridge surface of the receiving member 210. The air inlet 220 may connect the channel 110 of the main body 100 to the inside of the receiving member 210. When the user applies a suction force to the mouthpiece 120, the inhalable composition A may be discharged from the receiving member 210 to the channel 110 through the air outlet 230.

For example, the flow rate of air inhaled through the air inlet 220 or the air outlet 230 may be 1 to 60 lpm. Preferably, the flow rate of air may be 1.67 lpm.

In addition, a mesh net may be provided, for example, in the air inlet 220 or the air outlet 230.

As described above, since the cartridge 200 is coupled to the main body 100, the inhalable composition A may be transferred to the mouthpiece 120 through the channel 110, and thus, the user may inhale the inhalable composition A into the body.

In addition, the cartridge 200 may be detachably coupled to the first surface. Therefore, when the inhalable composition A contained in the cartridge 200 is exhausted, a new cartridge 200 may be used as a replacement and mounted on the main body 100. Here, the cartridge 200 may be fastened by magnets or in a fitting manner.

For example, the inhaler 10 according to an embodiment may be provided with a plurality of fastening members in each of the main body 100 and the cartridge 200, as shown in FIGS. 4A and 4B. The fastening members may be arranged adjacent to the first surface of the main body 100. In addition, the fastening members may be arranged on one side of the cartridge 200 at a position corresponding to the fastening members arranged on the main body 100. The fastening members may be provided as a magnetic structure, such as a magnet (neodymium, etc.). The fastening members of the main body 100 and the fastening members of the cartridge 200 may be provided with magnets having different polarities and may be detachably coupled to each other.

Furthermore, the cartridge 200 may be opened and closed by the cartridge cover 300. Specifically, the cartridge cover 300 may open and close the air inlet 220 or the air outlet 230 of the cartridge 200.

The cartridge cover 300 may be connected to surround the main body 100 and the cartridge 200 and may be slidably moved in the longitudinal direction along the side surface of the main body 100. When the cartridge cover 300 moves in the longitudinal direction, the cartridge 200 may be opened, and when the cartridge cover 300 moves in a direction opposite to the longitudinal direction, the cartridge 200 may be closed.

Referring to FIG. 2, the cartridge cover 300 may include a cover member 310 and a protruding member 320.

The cover member 310 may be arranged to surround the main body 100 and the cartridge 200. For example, the cover member 310 may surround the plurality of side surfaces of the main body 100 and the first cartridge surface of the cartridge 200. Here, the cover member 310 may be formed to have a longitudinal length that is greater than a longitudinal length of the cartridge. Accordingly, the cover member 310 may simultaneously surround the main body 100 and the cartridge 200, as shown in FIG. 1. In addition, the longitudinal length of the cartridge 200 may be formed to be 5 to 15 millimeters (mm).

The protruding member 320 may protrude from an inner side surface of the cover member 310 toward the main body 100, as shown in FIG. 2. For example, each protruding member 320 may protrude toward the left side surface and the right side surface of the main body 100. This protruding member 320 may be inserted into the slit 130 of the main body 100. The protruding member 320 may move along the slit 130.

The cartridge cover 300 may further include an elastic member (not shown).

The elastic member may be arranged between the cover member 310 and the main body 100. For example, one end of the elastic member may be fixed to the cover member 310 and the other end may be fixed to the main body 100. The elastic member may be provided as a torsion spring, for example. The elastic member may be compressed when the cover member 310 is pressed in the longitudinal direction and decompressed when the cover member 310 is pressed in the direction opposite to the longitudinal direction. Accordingly, the cartridge cover 300 may be opened and closed in a semi-automatic sliding manner.

The cartridge cover 300 including the aforementioned configuration may open the air inlet 220 or the air outlet 230 of the cartridge 200 by moving in the longitudinal direction along the slit 130 and may close the air inlet 220 or the air outlet 230 of the cartridge 200 by moving in the direction opposite to the longitudinal direction.

As a result, the user may open and close the air inlet 220 or the air outlet 230 of the cartridge 200 by sliding the cartridge cover 300 to inhale the inhalable composition A. In addition, the user may also move the cartridge cover 300 to replace the cartridge 200.

Referring to FIGS. 3A and 3B, the air inlet 220 of the cartridge 200 may be formed to extend in the longitudinal direction along the first cartridge surface.

FIG. 3A illustrates the inhaler 10 in which the air inlet 220 is closed by the cartridge cover 300. When the cartridge cover 300 is in a closed position, the air inlet 220 may not be in communication with the outside. Therefore, air or foreign substances from the outside may be prevented from flowing into the inhalable composition A by the cartridge cover 300.

When the cartridge cover 300 slides in a direction of an arrow of FIG. 3B, that is, in the longitudinal direction, the air inlet 220 of the cartridge 200 may be opened and exposed to the outside. Here, an open area of the air inlet 220 may gradually increase depending on a sliding movement distance of the cartridge cover 300. Therefore, the open area of the air inlet 220 may be adjusted depending on the movement distance of the cartridge cover 300.

Furthermore, even when the cartridge cover 300 covers a front surface of the air inlet 220, as shown in FIG. 3A, it may be possible to inhale the inhalable composition A through a pore due to mechanical tolerances between the main body 100, the cartridge 200, and the cartridge cover 300. For smooth inhalation, the user may completely open the air inlet 220 by sliding the cartridge cover 300 in the longitudinal direction as much as possible.

Referring to FIGS. 4A and 4B, the inhaler 10 according to an embodiment may further include the cartridge door 400.

The cartridge door 400 may be disposed between the main body 100 and the cartridge 200. The cartridge door 400 may be opened and closed by interworking with the cartridge cover 300. When the cartridge cover 300 moves in the longitudinal direction, the cartridge door 400 may connect the cartridge 200 to the channel 110, and when the cartridge cover 300 moves in the direction opposite to the longitudinal direction, the cartridge door 400 may operate to isolate the cartridge 200 from the channel 110

Specifically, the cartridge door 400 may include a door member 410 and a roller member 420.

The door member 410 may be arranged inside the main body 100. The door member 410 may be arranged parallel to the first surface. The door member 410 may be arranged adjacent to the air outlet 230 of the cartridge 200. The door member 410 may be formed, for example, in a flat shape. The door member 410 may also be provided, for example, as a mesh net. At least one end of the door member 410 may be connected to the cartridge cover 300.

The roller member 420 may be arranged inside the main body 100 so as to be in contact with one surface of the door member 410. The roller member 420 may change a direction of the door member 410 to a direction perpendicular to the first surface.

As shown in FIG. 4A, a portion of the door member 410 may be parallel to the first surface, and the remaining portion of the door member 410 may be bent by the roller member 420 and arranged parallel to the lower side surface of the main body 100. Here, as described above, since one end of the door member 410 is connected to the cartridge cover 300, the door member 410 may also slide along the sliding movement of the cartridge cover 300.

Referring to FIG. 4B, when the cartridge cover 300 slides in a direction of an arrow, that is, in the longitudinal direction, one end of the door member 410 may slide along the lower side surface of the main body 100. Accordingly, an area of the door member 410 arranged parallel to the first surface may decrease, and an area of the door member 410 in which the direction is changed to be parallel to the lower side surface may increase.

In contrary, when the cartridge cover 300 slides in the direction opposite to the longitudinal direction, the area of the door member 410 parallel to the lower side surface may decrease, and the area of the door member 410 in which the direction is changed to be parallel to the first surface may increase.

Referring again to FIGS. 4A and 4B, the air outlet 230 of the cartridge 200 may be formed to extend transversely across the longitudinal direction along the second cartridge surface.

FIG. 4A illustrates the inhaler 10 in a state in which the air outlet 230 is closed by the cartridge door 400. When the cartridge door 400 is in a closed position, the air outlet 230 may not be in communication with the channel 110. Accordingly, the inhalable composition A may be prevented from flowing out into the channel 110 by the cartridge door 400.

Referring again to FIGS. 5A and 5B, FIG. 5A illustrates the door member 410 of FIG. 4A. As shown in FIG. 4A, when the cartridge cover 300 is in the closed position, the door member 410 of the cartridge door 400 may cover the air outlet 230. Accordingly, the inhalable composition A may be isolated from the channel 110.

When the cartridge door 400 is opened as the cartridge cover 300 slides in the direction of the arrow of FIG. 4B, that is, in the longitudinal direction, the air outlet 230 of the cartridge 200 may be connected to the channel 110. Here, the open area of the air outlet 230 may gradually increase according to the sliding movement distance of the door member 410.

Referring again to FIGS. 5A and 5B, FIG. 5B illustrates the door member 410 of FIG. 4B. As shown in FIG. 4B, when the cartridge cover 300 slides, the door member 410 may move traversely. Accordingly, the inhalable composition A may be exposed to the channel 110. The door member 410 may be provided as, for example, a mesh.

Therefore, the open area of the air outlet 230 may be adjusted according to the movement distance of the door member 410. In addition, as described above, the door member 410 may slide by interworking with the cartridge cover 300. As a result, the open area of the air outlet 230 may be adjusted according to the movement distance of the cartridge cover 300.

As described above, the inhaler 10 according to an embodiment may easily open and close the cartridge 200 using the cartridge cover 300 and the cartridge door 400. In addition, the inhaler 10 according to an embodiment may have the cartridge cover 300 and the cartridge door 400, thereby having the effect of preventing leakage of the inhalable composition A and preventing introduction of foreign substances.

While the embodiments of the present disclosure have been described above with reference to specific components, and limited embodiments and drawings, the above descriptions are merely for better understanding of the present disclosure, and it will be apparent to one of ordinary skill in the art that various changes in form and details may be made in these embodiments without departing from the spirit and scope of the claims and their equivalents. For example, suitable results may be achieved if the described techniques are performed in a different order and/or if components in a described system, architecture, device, or circuit are combined in a different manner and/or replaced or supplemented by other components or their equivalents. Accordingly, the scope of the present disclosure is defined not by the detailed description, but by the claims and their equivalents, and all variations within the scope of the claims and their equivalents are to be construed as being included in the disclosure.

## Claims

1. An inhaler comprising:
a main body that comprises a first surface, a second surface facing the first surface and having a mouthpiece, and a plurality of side surfaces connecting the first surface to the second surface and has a channel formed inside in a longitudinal direction from the first surface toward the second surface;
a cartridge coupled to the first surface of the main body and containing an inhalable composition; and
a cartridge cover surrounding the main body and the cartridge and slidably coupled along a side surface of the main body in the longitudinal direction;
wherein the cartridge cover moves in the longitudinal direction to open the cartridge or moves in a direction opposite to the longitudinal direction to close the cartridge.

2. The inhaler of claim 1, further comprising:
a cartridge door that is arranged between the main body and the cartridge and opens and closes by interworking with the cartridge cover,
wherein the cartridge door connects the cartridge to the channel when the cartridge cover moves in the longitudinal direction, and
wherein the cartridge door isolates the cartridge from the channel when the cartridge cover moves in the direction opposite to the longitudinal direction.

3. The inhaler of claim 2, wherein
the cartridge door comprises:
a door member in a flat shape arranged parallel to the first surface inside the main body; and
a roller member arranged to be in contact with one surface of the door member and changing a direction of the door member to a direction perpendicular to the first surface;
wherein the door member has at least one end connected to the cartridge cover to slide.

4. The inhaler of claim 3, wherein,
when the cartridge cover moves in the longitudinal direction, the door member has an area arranged parallel to the first surface that decreases and an area converted in the direction perpendicular to the first surface that increases, and
when the cartridge cover moves in the direction opposite to the longitudinal direction, the door member has the area arranged parallel to the first surface that increases and the area converted in the direction perpendicular to the first surface that decreases.

5. The inhaler of claim 1, wherein
the cartridge comprises:
a receiving member that comprises a first cartridge surface parallel to one of the plurality of side surfaces and a second cartridge surface perpendicular to the first cartridge surface and in contact with the first surface and contains the inhalable composition therein;
an air inlet formed on the first cartridge surface of the receiving member; and
an air outlet formed on the second cartridge surface of the receiving member;
wherein the air inlet or the air outlet is opened when the cartridge cover moves in the longitudinal direction and closed when the cartridge cover moves in the direction opposite to the longitudinal direction.

6. The inhaler of claim 5, wherein
the air inlet is formed to extend in the longitudinal direction along the first cartridge surface, and
the cartridge cover controls an open area of the air inlet according to a sliding movement distance in the longitudinal direction.

7. The inhaler of claim 5, wherein
the air outlet is formed to extend transversely across the longitudinal direction along the second cartridge surface, and
the cartridge cover controls an open area of the air outlet according to a sliding movement distance in the longitudinal direction.

8. The inhaler of claim 1, wherein
the cartridge cover comprises:
a cover member arranged to surround the main body and a side surface of the cartridge; and
a protruding member protruding inwardly of the cover member and inserted into the side surface of the main body; and
a longitudinal length of the cover member is greater than a longitudinal length of the cartridge.

9. The inhaler of claim 8, wherein
the cartridge cover further comprises an elastic member arranged between the cover member and the main body, and
the elastic member is compressed when the cover member is pressed in the longitudinal direction and decompressed when the cover member is pressed in the direction opposite to the longitudinal direction.

10. The inhaler of claim 1, wherein
a slit extending along the longitudinal direction is formed on each of two facing side surfaces among the side surfaces the main body, and the cartridge cover is coupled to the slit and slides along the slit.

11. The inhaler of claim 1, wherein
the cartridge is detachably attached to the first surface by magnets or in a fitting manner.
